Europäisches Patentamt

**European Patent Office**

Office européen des brevets

(19)

(11) Numéro de publication: **0 056 877**
**B1**

(12) **FASCICULE DE BREVET EUROPEEN**

(45) Date de publication du fascicule du brevet: **22.05.85**

(21) Numéro de dépôt: **81200071.9**

(22) Date de dépôt: **22.01.81**

(51) Int. Cl.⁴: **A 46 B 15/00,** A 61 B 1/24

(54) Appareil pour soins buccaux.

(43) Date de publication de la demande:
**04.08.82 Bulletin 82/31**

(45) Mention de la délivrance du brevet:
**22.05.85 Bulletin 85/21**

(84) Etats contractants désignés:
**CH DE FR GB IT LI**

(56) Documents cités:
**CH-A- 598 801**
**DE-A-2 653 264**
**FR-A-1 538 649**
**FR-A-2 323 354**
**GB-A-2 040 170**
**US-A-1 473 357**
**US-A-2 841 806**
**US-A-3 261 978**
**US-A-3 667 454**
**US-A-4 195 329**

(73) Titulaire: **LES PRODUITS ASSOCIES LPA S.A.**
**39, rue Peillonnex**
**CH-1225 Chene-Bourg (CH)**

(72) Inventeur: **Woog, Philippe Guy E.**
**"Le Séquoia" Chemin Botterel**
**CH-1222 Vesenaz (CH)**

(74) Mandataire: **Jörchel, Dietrich R.A. et al**
**c/o BUGNION S.A. Conseils en Propriété**
**Industrielle 10, route de Florissant Case postale**
**375**
**CH-1211 Genève 12 Champel (CH)**

Courier Press, Leamington Spa, England.

EP 0 056 877 B1

## Description

La présente invention a pour objet un appareil pour soins buccaux comprenant un boîtier-poignée, en particulier de forme allongée, destiné à fixer une brosse à dents et dans lequel est monté un moteur électrique d'entraînement de la brosse à dents et une source de lumière alimentée par la même source de tension qui alimente le moteur électrique actionnable par un interrupteur et montée dans un évidement du boîtier-poignée.

Les brosses à dents électriques sont connues depuis de nombreuses années et leur utilisation est maintenant entrée dans les moeurs, leur efficacité et leur maniabilité constituant leur atout principal. Les brosses à dents les plus courantes comportent un boîtier-poignée allongé dans lequel est monté le moteur électrique destiné à entraîner, par exemple d'un mouvement oscillant, un arbre qui fait saillie à l'extrémité du boîtier et sur lequel se fixe la tige des brosses à dents interchangeables.

Il est également connu depuis quelques temps des appareils ou dispositifs qui permettent à tout un chacun de tester à la maison ses dents et ses gencives, afin de vérifier si elles sont recouvertes de plaque dentaire. Comme on le sait depuis quelques années, la plaque dentaire est une pellicule, normalement invisible, qui se forme sur les dents composée en particulier de bactéries, et elle constitue l'un des principaux facteurs de la détérioration des dents, c'est pourquoi il est important de vérifier régulièrement l'état de la bouche afin de détecter la présence de cette plaque. Le procédé le plus utilisé pour rendre cette plaque dentaire visible est d'enduire les zones à tester avec une solution fluorescente et exciter cette solution au moyen de lampes émettant un spectre lumineux qui répond à la solution fluorescente. Il a été démontré que le matériau fluorescent adhérait seulement sur les parties des dents recouvertes par cette plaque, mais non sur les parties propres et saines. Il a également été constaté que ce matériau fluorescent adhérait aussi sur les parties entartrées, le tartre étant formé par une minéralisation de la plaque, sur les parties attaquées par la carie dentaire et même sur les parties affectées des gencives.

Ainsi en appliquant une couche de matériau fluorescent sur les dents et les gencives, et en excitant ce matériau à l'aide d'une lampe appropriée, chaque particulier peut tester l'état de sa bouche.

Différents dispositifs ont été proposés pour mettre en évidence cette plaque dentaire, notamment le dispositif décrit dans le brevet suisse du déposant No. 598 801 comprenant une lampe-test montée dans un boîtier, alimentée par une pile, un distributeur de produit fluorescent assemblé au boîtier, un filtre adapté à la solution fluorescente utilisée de façon à faire transmettre essentiellement les longueurs d'onde de la lumière nécessaires à l'excitation fluorescente et absorber essentiellement la gamme du spectre correspondant à la lumière fluorescente. Une lampe de poche simple peut également convenir à condition de la munir également d'un filtre approprié. Donc si un consommateur veut suivre une hygiène dentaire rigoureuse il doit avoir sous la main, dans sa salle de bains, d'une part la brosse à dents électrique qui lui assure un nettoyage efficace des dents et d'autre part le matériel pour tester l'état de sa bouche: matériau fluorescent et lampe-test. Ceci représente beaucoup d'accessoires qui encombrent la salle de bain et d'autre par risquent de s'égarer.

Dans le brevet US—A—3.667.454, il est décrit une brosse à dents vibrante dont la tige est munie d'un tube émettant, à l'emplacement des soies, des radiations ultraviolettes.

Un autre brevet US—A—3.261.978 décrit une brosse à dents munie dans son manche d'une source de lumière ultraviolette laquelle est transmise à un filtre disposé dans la tête de la brosse, à l'opposé des soies.

La présente invention se propose de réaliser un appareil combiné qui d'une part, permet de diminuer avantageusement le nombre d'accessoires nécessaires à une hygiène dentaire rigoureuse et d'autre part, par une localisation avantageuse de la source de lumière et d'une configuration spéciale du boîtier, peut avoir une position de repos peu encombrante.

A cet effet, l'appareil est caractérisé par la revendication 1.

L'utilisateur ayant toujours sa brosse à dents à portée de main dans la salle de bain, il aura donc en même temps sous la main la lampe-test, ce qui l'incitera à vérifier plus souvent l'état de sa bouche. Cette combinaison ingénieuse: brosse à dents et lampe-test peut être fabriquée pratiquement pour le même prix qu'une brosse à dents électrique simple, ce qui est avantageux pour le consommateur et lui permet d'éviter les frais d'une lampe spéciale indépendante.

En outre, cette brosse à dents peut, comme cela est connu, être suspendue à un support mural approprié ou, grâce à son fond plat, elle peut être simplement posée verticalement sur la tablette de la salle de bain ce qui la rend moins encombrante que lorsqu'elle est posée horizontalement en prenant appui sur sa périphérie, position dans laquelle elle risque en outre de rouler.

Préférablement, l'appareil est également équipé d'un récipient soit amovible, soit incorporé au boîtier, pour contenir le matériau fluorescent.

Des formes d'exécution préférées de l'invention résultent des autres revendications.

La présente invention va maintenant être décrite, à titre d'exemple, en référence aux dessins annexés dans lesquels:

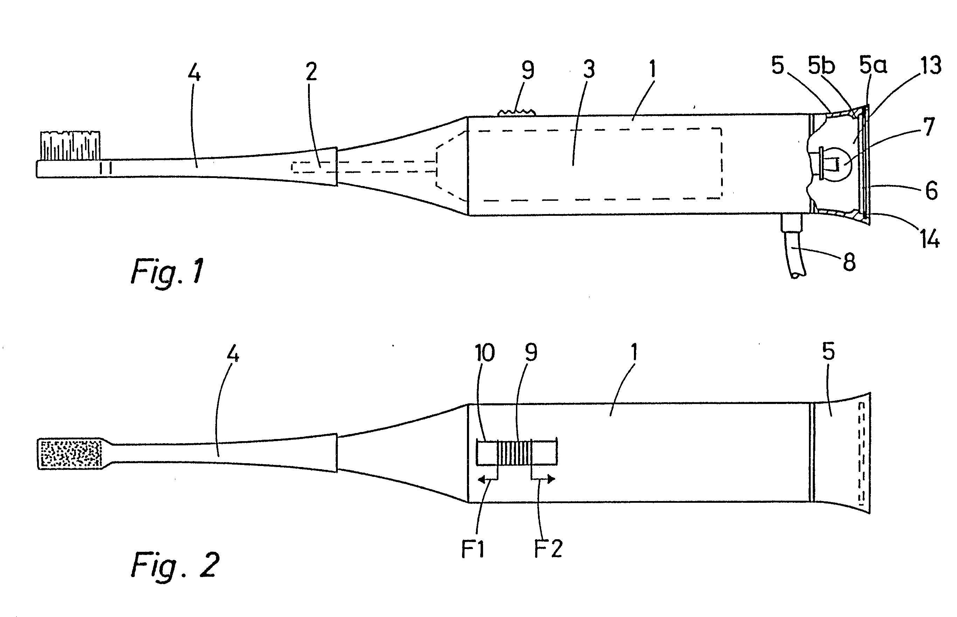

la figure 1 est une vue en élévation d'une brosse à dents selon l'invention avec coupe partielle de l'extrémité postérieure du boîtier.

La figure 2 en est une vue de dessus, montrant la face munie de l'interrupteur.

La figure 3 est une vue d'une variante d'exécution des interrupteurs de l'appareil avec, en traits mixtes, un récipient amovible destiné à contenir le matériau fluorescent.

La figure 4 est une variante d'exécution de l'appareil.

L'appareil comprend un boîtier-poignée allongé 1 à l'extrémité antérieure duquel dépasse, de la manière connue, un arbre 2 prévu pour être entraîné selon un mouvement oscillant par un moteur électrique 3 incorporé dans le boîtier 1. Sur cet arbre 2 se monte la tige 4 des brosses à dents interchangeables. L'extrémité postérieure 5 du boîtier 1 a une forme évasée et se termine par un rebord 5a sur lequel l'appareil peut prendre appui lorsqu'il est posé sur un support plan. A distance de l'extrémité de ce rebord 5a est monté un filtre dichroïque 6 qui est, par exemple, retenu entre un épaulement 5b de la paroi latérale du boîtier et un anneau 14. Ce filtre dichroïque 6, qui est de cette manière en retrait et donc protégé par le rebord 5a, permet de laisser passer les rayons lumineux, de longueurs d'ondes choisies venant d'une ampoule incandescente 7 fixée dans un évidement 13 du boîtier 1 et dirigée vers le filtre. Ce filtre 6 est assorti au matériau fluorescent utilisé pour transmettre essentiellement les rayons provenant de la source de lumière nécessaires à l'excitation fluorescente et absorber essentiellement la gamme du spectre correspondant à la lumière fluorescente.

Pour remplacer l'ampoule 7, la partie postérieure 5 de l'appareil peut être séparée de la partie principale 1, et elle est à cet effect par exemple vissée sur ledit boîtier. Une autre solution consiste d'avoir simplement l'anneau 14 amovible.

Sur la forme d'exécution représentée, le moteur électrique 3 ainsi que l'ampoule 7 sont alimentés par le secteur au moyen d'un cordon électrique 8 et un interrupteur unique 9, placé sur la paroi latérale du boîtier 1, permet de commander la mise en marche et l'arrêt soit de la brosse à dents soit de l'ampoule incandescente 7. L'interrupteur 9 est disposé dans une fenêtre 10 de la paroi latérale du boîtier 1 de telle façon que dans la position centrale représentée, il se trouve en position de repos, donc ni l'ampoule ni la brosse ne sont actionnées, que dans la position limite vers la gauche du dessin selon la flèche F1, c'est la brosse à dents qui se trouve actionnée et dans la position limite vers la droite du dessin, selon la flèche F2, l'ampoule incandescente 7 est mise sous tension et l'appareil peut être utilisé pour le test lumière.

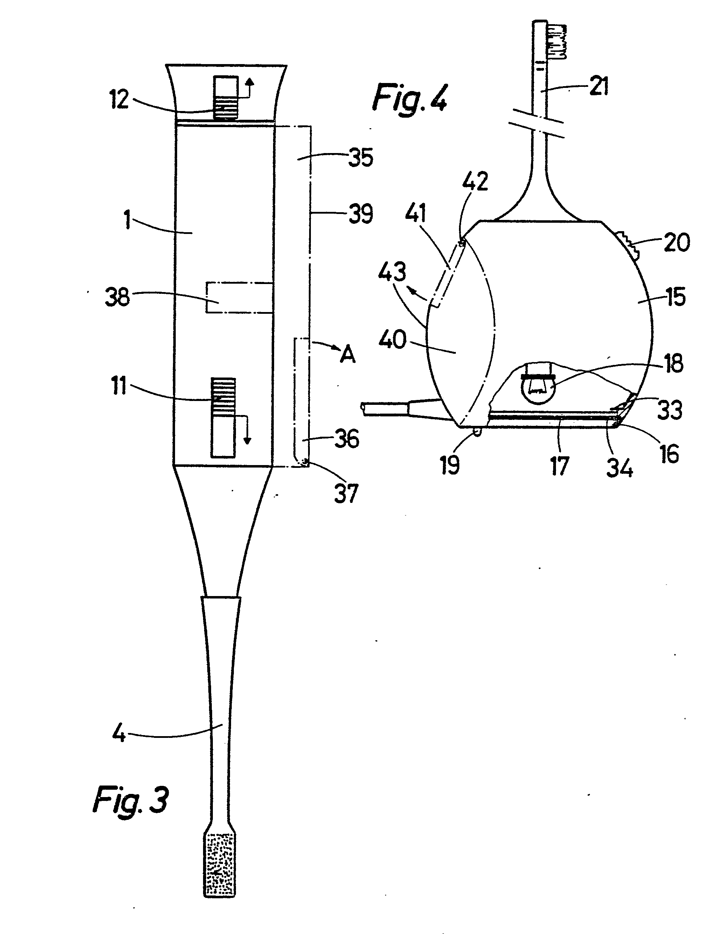

Selon une autre variante d'exécution de l'appareil selon figure 3, les interrupteurs 11 et 12 sont séparés l'un de l'autre, l'interrupteur de la brosse à dents 11 se trouvant à l'extrémité du boîtier dirigée vers la brosse 4 tandis que l'interrupteur de l'ampoule 7 se trouve à l'extrémité postérieure.

Dans la forme d'exécution représentée, le moteur électrique 3 et l'ampoule 7 sont alimentés par le secteur, préférablement via un transformateur, mais l'appareil peut naturellement être conçu pour fonctionner par pile, rechargeable ou non, une ou plusieurs piles étant alors emmagasinées dans un évidement approprié du boîtier ou sur un support extérieur au boîtier. On peut également concevoir que l'ampoule seule est alimentée par une pile alors que le moteur électrique marche sur le secteur.

La position de l'ampoule 7 et du filtre dichroïque 6 n'est bien entendue pas limitée au fond du boîtier, bien que cet emplacement semble être le plus pratique afin que la lumière puisse être dirigée efficacement vers les parties buccales à tester. N'importe quelle autre partie de la paroi latérale du boîtier 1 peut être munie de ce filtre 6. Par ailleurs, un réflecteur dichroïque, non représenté, peut être monté derrière l'ampoule afin d'amplifier le rayonnement de celle-ci.

En outre, on pourrait prévoir une source de lumière émettant seulement des longueurs d'ondes courtes et la radiation pourrait être au moins approximativement monochromatique. La source de lumière la mieux appropriée à cet effet est celle fournie par les diodes lumineuses ou électroluminescentes et l'ampoule pourrait donc être remplacée par une ou plusieurs diodes lumineuses disposées côte à côte.

D'autres variantes d'exécution pourraient être prévues, notamment l'extrémité de la partie postérieure 5 du boîtier pourrait avoir une autre forme qu'évasée, par exemple simplement cylindrique.

Sur la figure 3 est également représentée schématiquement et en traits mixtes une autre variante de l'appareil, à savoir sa combinaison avec un récipient 35 contenant le matériau fluorescent, par exemple de la fluorescéine. Ce récipient 35 est formé avec une paroi qui s'adapte à la forme cylindrique du boîtier 1 et est fixé amoviblement sur ledit boîtier par un clips 38. Il est en outre muni d'un tube de sortie 36 basculable selon la flèche A autour d'un axe 37 et qui, en position escamotée, ferme l'ouverture du récipient, comme décrit dans le brevet suisse No. 598 801 du déposant. La paroi extérieure 39 est semi-rigide sur toute ou une partie de sa surface afin que l'utilisateur puisse extraire le matériau du récipient par simple pression sur cette paroi.

Sur la figure 4 est illustrée une autre variante d'exécution de l'appareil. Dans ce cas, le boîtier 15 a une forme générale sensiblement ovoïde, mais avec des extrémités tronquées, et il peut être posé sur un support, lorsqu'il n'est pas utilisé, en appui sur sa face inférieure plane constituée par un rebord annulaire 16. Le filtre dichroïque 17 est monté dans le fond du boîtier 15, à distance du rebord annulaire 16 de manière à ne pas toucher la surface d'appui sur laquelle est posé l'appareil. Le filtre 17 est fixé entre un épaulement 33 sur la paroi latérale du boîtier et un anneau de retenue 34, il est de cette manière démontable pour rendre l'ampoule 18 accessible.

Comme dans la forme d'exécution précédente, la source de lumière est constituée par une ampoule à incandescence 18 ou des diodes lumineuses. L'interrupteur de la source de lumière est constitué par un contact à ressort 19 monté dans le fond du boîtier et dépassant légèrement du niveau du fond à l'état fermé. Ce contact est conçu de manière à couper le circuit d'alimentation de la lampe 18 quand l'appareil est en position de repos, en appui sur un support, et à fermer le circuit, donc allumer l'ampoule, quand l'appareil est écarté de son support. Donc avec cette forme d'exécution, la lampe s'allume automatiquement dès que l'utilisateur prend l'appareil en main. Un autre interrupteur 20 est prévu pour la commande du moteur de la brosse à dents 21, cet interrupteur étant situé, de préférence, sur la paroi latérale du boîtier.

Par ailleurs, cette invention peut s'appliquer en principe également à une brosse à dents entraînée par un moteur hydraulique, dans ce cas la source de lumière est alimentée par une batterie. En outre, cette invention est aussi applicable à un appareil combiné qui est non seulement adapté pour entraîner une brosse à dents, mais pour alimenter une buse de giclage fixée à la tête.

## Revendications

1. Appareil pour soins buccaux comprenant un boîtier-poignée (1; 15), en particulier de forme allongée, destiné à fixer une brosse à dents, et dans lequel est monté un moteur électrique (3) d'entraînement de la brosse à dents et une source de lumière (7; 18) alimentée par la même source de tension qui alimente le moteur électrique (3) actionnable par un interrupteur (9; 10, 12) et montée dans un évidement (13) du boîtier-poignée (1), caractérisé par le fait que l'évidement (13) qui contient la source de lumière (7; 18) se trouve à l'extrémité postérieure du boîtier-poignée (1; 15) et est recouvert par un filtre dichroïque (6; 17) adapté pour transmettre essentiellement le rayonnement d'excitation fluorescente d'un matériau fluorescent appliqué sur des parties buccales et servant à tester leur état, ce filtre étant disposé préférablement en retrait par rapport au fond du boîtier-poignée (1; 15), ce fond est constitué par un rebord annulaire (5a; 16) constituant une surface d'appui sur laquelle l'appareil prend appui lorsqu'il repose sur une surface plane.

2. Appareil selon la revendication 1, caractérisé par le fait que la source de tension est constituée par le réseau, auquel il est préférablement connecté via un transformateur, ou une batterie, préférablement rechargeable et montée dans le boîtier-poignée ou sur un support extérieur.

3. Appareil selon la revendication 1 ou 2, caractérisé par le fait que le même interrupteur (9) sert à commander alternativement le moteur électrique (3) pour l'entraînement de la brosse à dents, et la source de lumière (7).

4. Appareil selon la revendication 1 ou 2, caractérisé par le fait que l'interrupteur (12) de commande de la source de lumière (7) est indépendant de l'interrupteur (11) de commande du moteur électrique (3).

5. Appareil selon la revendication 4, caractérisé par le fait que l'interrupteur est un contacteur à ressort (19) monté sur le fond du boîtier-poignée, qui, dans l'état de fermeture du circuit de la source de lumière (18), dépasse du niveau du fond et est agencé de manière à être ouvert quand l'appareil repose sur un support et à être mis à l'état fermé quand l'appareil est soulevé de son support.

6. Appareil selon l'une quelconque des revendications 1 à 5, caractérisé par le fait que le filtre dichroïque est amovible, notamment par le démontage d'un anneau de retenue (34), ou par dévissage de la partie du boîtier-poignée sur laquelle il est monté.

7. Appareil selon l'une quelconque des revendications 1 à 6, caractérisé par le fait qu'il est prévu un récipient (35, 40) monté préférablement amoviblement sur ou incorporé dans le boîtier, destiné à contenir ledit matériau fluorescent, en particulier de la fluorescéine.

## Patentansprüche

1. Mundpflegegerät mit einem grifförmigen Gehäuse (1; 15), insbesondere von gestreckter Form, welches zur Befestigung einer Zahnbürste eingerichtet ist und in welchem ein Elektromotor (3) zum Antrieb der Zahnbürste und eine Lichtquelle (7; 18) installiert sind, welche von der gleichen Spannungsquelle gespeist wird, die den Elektromotor (3) speist, durch einen Schalter (9; 10, 12) betätigbar ist und in einer Ausnehmung (13) des grifförmigen Gehäuses (1) montiert ist, dadurch gekennzeichnet, dass die Ausnehmung (13), welche die Lichtquelle (7; 18) enthält, sich am rückwärtigen Ende des griffartigen Gehäuses (1; 15) befindet und durch ein dichroitisches Filter (6; 17) abgedeckt ist, welches dazu eingerichtet ist, im wesentlichen die Fluoreszenzanregungsstrahlung eines fluoreszierenden Materials durchzulassen, das auf Bereiche des Mundes aufgebracht wurde und dazu dient, deren Zustand zu prüfen, und dass dieses Filter vorzugsweise in Bezug auf den Boden des grifförmigen Gehäuses (1; 15) nach innen versetzt ist, wobei dieser Boden durch eine ringförmigen Rand (5a; 16) gebildet wird, der eine Auflagefläche darstellt, auf welcher das Gerät ruht, wenn es auf eine ebene Fläche abgesetzt wird.

2. Gerät nach Anspruch 1, dadurch gekennzeichnet, dass die Spannungsquelle durch das Netz, an das es vorzugsweise über einen Transformator anschliessbar ist, oder durch eine vorzugsweise aufladbare Batterie, welche im grifförmigen Gehäuse oder auf einem äusseren Trägerteil installiert ist, gebildet wird.

3. Gerät nach Anspruch 1 oder 2, dadurch gekennzeichnet, dass derselbe Schalter (9) alternativ zur Steuerung des Elektromotors (3) für den Zahnbürstenantrieb oder der Lichtquelle (7) dient.

4. Gerät nach Anspruch 1 oder 2, dadurch gekennzeichnet, dass der Schalter (12) zur Steuerung der Lichtquelle (7) unabhängig ist vom Schalter (11) zur Steuerung des Elektromotors (3).

5. Gerät nach Anspruch 4, dadurch gekennzeichnet, dass der Schalter ein federbelasteter Kontakt (19) ist, der auf dem Boden des grifförmigen Gehäuses installiert ist und welcher, wenn et sich in dem den Stromkreis der Lichtquelle (8) schliessenden Zustand befindet, das Niveau des Bodens überragt und derart angeordnet ist, dass er sich im offenen Zustand befindet, wenn das Gerät auf einer Unterlage ruht, und im geschlossenen Zustand befindet, wenn das Gerät von seiner Unterlage abgehoben ist.

6. Gerät nach einem der Ansprüche 1 bis 5, dadurch gekennzeichnet, dass das dichroitische Filter abnehmbar ist, insbesondere durch Demontage eines Halterings (34) oder durch Abschrauben des Teils des grifförmigen Gehäuses, an welchem es montiert ist.

7. Gerät nach einem der Ansprüche 1 bis 6, dadurch gekennzeichnet, dass ein zur Aufnahme des erwähnten fluoreszierenden Materials, insbesondere von Fluoreszein, bestimmter Behälter (35, 40) vorgesehen ist, der vorzugsweise abnehmbar auf dem oder im Gehäuse montiert ist.

**Claims**

1. Apparatus for mouth hygiene, comprising a handle-case (1, 5), in particular of elongated shape, being intended for the attachment of a toothbrush and incorporating an electric motor (3) for driving the toothbrush, and a light source (7; 18) energized by the same voltage source that feeds the electric motor (3), actuatable by a switch (9, 10; 11) and mounted in a recess (13) of the handle-case (1), characterized in that the recess (13) comprising the light source (7; 18) is located at the rear end of the handle-case (1; 15) and covered by a dichroic filter (6; 17) adapted to transmit essentially the fluorescence excitation radiation of a fluorescent material applied to the mouth areas and serving to test their condition, this filter being preferably recessed in relation to the bottom of the handle-case (1; 15), this bottom consisting of an annular edge (5a; 16) constituting a bearing surface on which the apparatus rests when laid upon a flat surface.

2. Apparatus according to claim 1, characterized in that the voltage source is constituted by the electric mains, to which the apparatus is preferably connected via a transformer, or by a battery, preferably a rechargeable battery, mounted in the handle-case or on an external support.

3. Apparatus according to any of claims 1 or 2, characterized in that the same switch (9) is serving for alternatively controlling the electric motor (3) for driving the toothbrush and the light source (7).

4. Apparatus according to any of claims 1 or 2, characterized in that the switch (12) controlling the light source (7) is independent of the switch (11) controlling the electric motor (3).

5. Apparatus according to claim 4, characterized in that the switch is a spring contact (19) mounted on the bottom which, when the circuit of the light source (18) is closed, projects from the bottom level and is so arranged as to be open when the apparatus rests on a support, and be closed when the apparatus is lifted off its support.

6. Apparatus according to any of claims 1 to 5, characterized in that the dichroic filter is detachable, notably by removing a retaining ring (34), or by unscrewing the part of the handle-case on which it is mounted.

7. Apparatus according to any of claims 1 to 6, characterized in that there is provided a container (35, 40) mounted preferably detachably on, or incorporated in, the case, adapted to contain said fluorescent material, notably fluorescein.

Fig. 1

Fig. 2

Fig. 4

Fig. 3